# EUROPÄISCHE PATENTSCHRIFT

(11) **EP 0 695 296 B1**
(45) Veröffentlichungstag und Bekanntmachung des Hinweises auf die Patenterteilung: **06.03.2002**
(21) Anmeldenummer: 94914369.7
(22) Anmeldetag: 14.04.1994
(51) Int. Cl.: C07D 239/60, C07D 239/52, C07D 333/24, C07D 307/46, C07C 69/22, A01N 43/54, C07D 405/12, C07D 409/12

(54) **3-ARYLOXY-CARBONSÄUREDERIVATE UND VERFAHREN ZU IHRER HERSTELLUNG**
3-ARYLOXY CARBOXYLIC ACID DERIVATIVES AND METHOD OF PREPARING THEM
DERIVES D'ACIDE 3-ARYLOXY CARBOXYLIQUE ET PROCEDE POUR PRODUIRE CES DERIVES

(30) Priorität: 23.04.1993 DE 4313413
(43) Veröffentlichungstag der Anmeldung: 07.02.1996
(73) Patentinhaber: BASF AKTIENGESELLSCHAFT, 67056 Ludwigshafen (DE)
(72) Erfinder: BAUMANN, Ernst, D-67373 Dudenhofen (DE); RHEINHEIMER, Joachim, D-67063 Ludwigshafen (DE); VOGELBACHER, Uwe, Josef, D-67071 Ludwigshafen (DE); BRATZ, Matthias, D-67346 Speyer (DE); MEYER, Norbert, D-68526 Ladenburg (DE); GERBER, Matthias, D-67117 Limburgerhof (DE); WESTPHALEN, Karl-Otto, D-67346 Speyer (DE); WALTER, Helmut, D-67283 Obrigheim (DE); RADEMACHER, Wilhelm, D-67117 Limburgerhof (DE)
(86) Internationale Anmeldenummer: EP9401156
(87) Internationale Veröffentlichungsnummer: WO9425443

(56) Entgegenhaltungen:
- EP-A- 0 098 892
- EP-A- 0 347 811
- EP-A- 0 415 384
- EP-A- 0 481 512
- EP-A- 0 517 215
- PATENT ABSTRACTS OF JAPAN vol. 15, no. 456 (C-0886)20. November 1991 & JP,A,03 193 796 (KUMIAI CHEM. IND. CO. LTD.) 23. August 1991
- CHEMICAL ABSTRACTS, vol. 119, no. 13, 27. September 1993, Columbus, Ohio, US; abstract no. 139254e, HARADA K ET AL 'Preparation of pyrimidinyloxy- and pyrimidinylthiobutyric acid derivatives as herbicides' Seite 883 ;Spalte 2 ; & JP,A,04 356 470 (UBE INDUSTRIES, LTD.;JAPAN) 10. Dezember 1992
- JOURNAL OF MEDICINAL CHEMISTRY Bd. 35, Nr. 17 , 21. August 1992 Seiten 3246 - 3253 LI R. ET AL. 'Synthesis, characterization, and Ca2+ antagonistic activity of diltiazem metabolites'
- AUSTRALIAN JOURNAL OF CHEMISTRY Bd. 45, Nr. 11 , 1992 Seiten 1833 - 1843 BROWN RFC ET AL. 'Reactions of methyl thre o-2-acetoxy-3-chloro-3-(4-methoxyphenyl)pr opanoate and methyl cis-2,3-epoxy-3-(4-met hoxyphenyl)propanoate with 3,5-dimethoxyphenol: potential routes to flavan-3-ols'
- CHEMICAL & PHARMACEUTICAL BULLETIN (TOKYO) Bd. 40, Nr. 8 , 1992 Seiten 2055 - 2061 YANAGISAWA H ET AL. 'Synthesis and antihypertensive activity of 3-acetoxy-2,3 -dihydro-5-[2-(dimethylamino)ethyl]-2-(4-m ethoxyphenyl)-1,5-benzothiazepin-4(5H)-one (Diltiazem) derivatives having substituents at the 8 position'
- CHROMATOGRAPHIA Bd. 35, Nr. 5-6 , März 1993 Seiten 305 - 310 NISHI H ET AL. 'Direct enantiomeric separation of racemic precursors to diltiazem hydrochloride and clentiazem maleate by HPLC on a chiral ovomucoid column'

## Beschreibung

Die vorliegende Erfindung betrifft 3-Aryloxy-Carbonsäurederivate der allgemeinen Formel I, 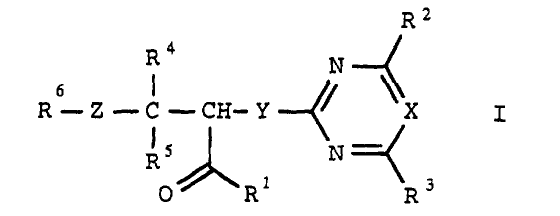 in der die Substituenten folgende Bedeutung haben:
- R¹: einen Rest OR¹⁰, worin R¹⁰ bedeutet:
i) Wasserstoff, ein Alkalimetallkation, das Äquivalent eines Erdalkalimetallkations, das Ammoniumkation oder ein organisches Ammoniumion;
ii) eine C₁-C₁₀-Alkylgruppe;
- R²: Methoxy;
- X: CR¹⁴, wobei R¹⁴ Wasserstoff bedeutet;
- R³: Methoxy;
- R⁴: Phenyl, das durch einen oder mehrere der folgenden Reste substituiert sein kann: Halogen, Nitro, Cyano, Hydroxy, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, C₁-C₄-Alkylamino oder C₁-C₄-Dialkylamino;
- R⁵: C₁-C₄-Alkyl;
- R⁶: Phenyl, das durch einen oder mehrere der folgenden Reste substituiert sein kann: Halogen, Nitro, Cyano, Hydroxy, Amino, C₁-C₄-Alkyl, C₁-C₄-Halogenalkyl, C₁-C₄-Alkoxy, C₁-C₄-Halogenalkoxy, Phenoxy, C₁-C₄-Alkylthio, C₁-C₄-Alkylamino oder C₁-C₄-Dialkylamino;
- Y: Sauerstoff;
- Z: Sauerstoff.

Im Stand der Technik, z.B. EP-A 347 811, EP-A 400 741,
EP-A 409 368, EP-A 481 512, EP-A 517 215 und in der älteren deutschen Anmeldung P 41 42 570 vom 21.12.91 werden ähnliche Carbonsäurederivate beschrieben. Unter anderem werden auch 3-Alkoxyderivate beschrieben, nicht jedoch 3-Aryloxy-Carbonsäurederivate. Die herbizide und/oder bioregulatorische Wirkung und Selektivität der bekannten Verbindungen ist jedoch nicht immer befriedigend.

Der Erfindung 1ag daher die Aufgabe zugrunde, Verbindungen mit verbesserter Selektivität und/oder biologischer Wirkung bereitzustellen.

Es wurde nun gefunden, daß die eingangs definierten 3-Aryloxy-Carbonsäurederivate ausgezeichnete herbizide und pflanzenwachstumsregulierende Eigenschaften haben.

Die Herstellung der erfindungsgemäßen Verbindungen geht aus von den Epoxiden IV, die man in allgemein bekannter Weise aus den Aldehyden bzw. Ketonen II, 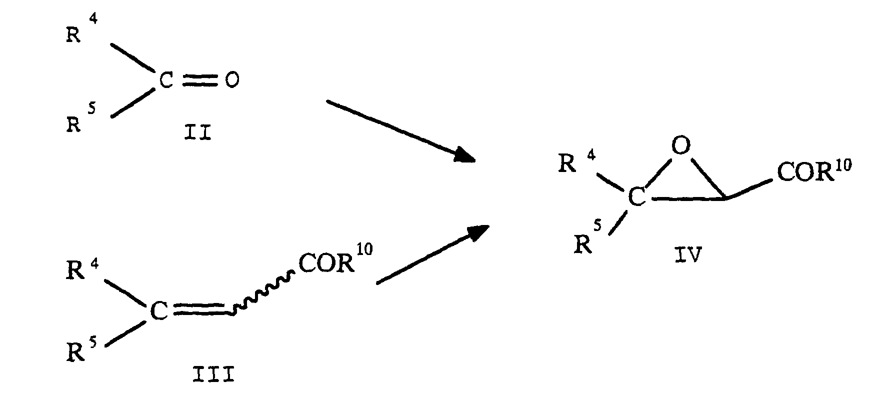 wie z. B. in J. March, Advanced Organic Chemistry, 2nd ed., 1983, S. 862 beschrieben, oder aus den Olefinen III, wie z. B. ibid, S. 750 beschrieben, erhält.

3-Aryl-oxy-Carbonsäurederivate der allgemeinen Formel VI können hergestellt werden, indem man die Epoxide der allgemeinen Formel IV mit oder Aryloxyverbindungen der allgemeinen Formel V, in der R⁶ und Z die in Anspruch 1 genannte Bedeutung haben, zur Reaktion bringt.

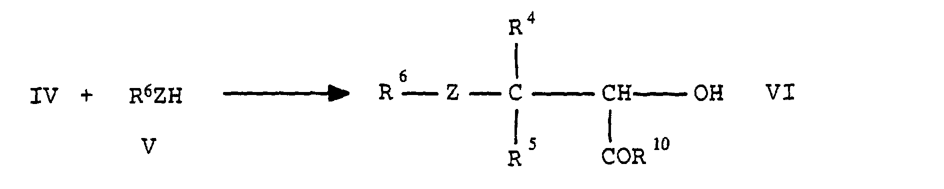

Dazu werden Verbindungen der allgemeinen Formel IV mit einem Überschuß der Verbindungen der Formel V, z.B. mit 1,2 bis 7, bevorzugt 2 - 5 Moläquivalenten V, auf eine Temperatur von 50 - 200°C, bevorzugt 80 - 150°C erhitzt. Die Reaktion kann auch in Gegenwart eines Verdünnungsmittels erfolgen. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide und Sulfone, wie zum Beispiel Dimethylsulfoxid und Sulfolan, und Basen, wie zum Beispiel Pyridin.

Wird ein Lösungsmittel verwendet, so erfolgt die Reaktion bevorzugt in einem Temperaturbereich zwischen 0°C und dem Siedepunkt des Lösungsmittels bzw. Lösungsmittelgemisches.

Die Gegenwart eines Reaktionskatalysators kann von Vorteil sein. Als Katalysatoren kommen dabei Säuren und Lewissäuren in Frage. Beispiele hierfür sind unter anderem Schwefelsäure, Salzsäure, Trifluoressigsäure, Bortrifluorid-Etherat und Titan(IV)-Alkoholate.

Die erfindungsgemäßen Verbindungen, in denen Y Sauerstoff bedeutet und die restlichen Substituenten die unter der allgemeinen Formel I angegebenen Bedeutung haben, können beispielsweise derart hergestellt werden, daß man die 3-Aryloxy-Carbonsäurederivate der allgemeinen Formel VI, in denen die Substituenten die angegebene Bedeutung haben, mit Verbindungen der allgemeinen Formel VII, in der R¹⁵ Halogen oder R¹⁶-SO₂- bedeutet, wobei R¹⁶ C₁-C₄-Alkyl, C₁-C₄-Halogenalkyl oder Phenyl sein kann, zur Reaktion bringt:

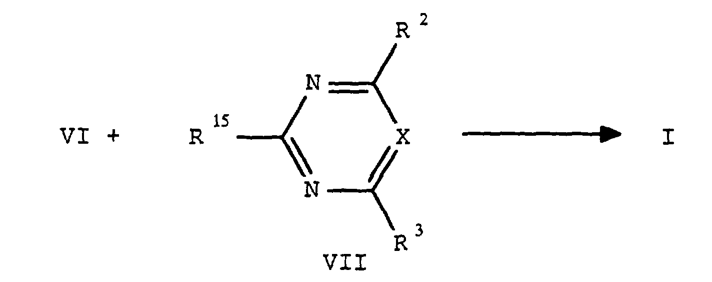

Die Reaktion findet bevorzugt in einem der oben genannten inerten Verdünnungsmittel unter Zusatz einer geeigneten Base in einem Temperaturbereich von Raumtemperatur bis zum Siedepunkt des Lösungsmittels statt.

Als Base kann ein Alkali- oder Erdalkalimetallhydrid wie Natriumhydrid, Kaliumhydrid oder Calciumhydrid, ein Carbonat wie Natrium- oder Kaliumcarbonat, ein Metallhydroxid wie Natrium- oder Kaliumhydroxid oder eine metallorganische Verbindung wie Butyllithium oder ein Alkaliamid wie Lithiumdiisopropylamid dienen.

Die erfindungsgemäßen Verbindungen, in denen Y Schwefel bedeutet und die restlichen Substituenten die unter der allgemeinen Formel I angegebene Bedeutung haben, können beispielsweise derart hergestellt werden, daß man 3-Aryloxy-Carbonsäurederivate der allgemeinen Formel VIII, die in bekannter Weise aus Verbindungen der allgemeinen Formel VI erhältlich sind und in denen die Substituenten die oben angegebene Bedeutung haben mit Verbindungen der allgemeinen Formel IX, in der R², R³ und X die unter der allgemeinen Formel I angegebene Bedeutung haben, zur Reaktion bringt.

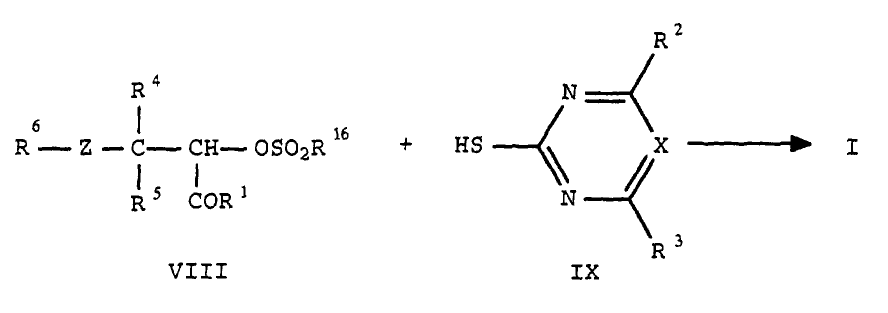

Die Reaktion findet bevorzugt in einem der oben genannten inerten Verdünnungsmittel unter Zusatz einer geeigneten Base in einem Temperaturbereich von Raumtemperatur bis zum Siedepunkt des Lösungsmittels statt.

Als Base können neben den oben genannten auch organische Basen wie Triethylamin, Pyridin, Imidazol oder Diazabicycloundecen dienen.

Verbindungen der Formel I können auch dadurch hergestellt werden, daß man von den entsprechenden Carbonsäuren, d. h. Verbindungen der Formel I, in denen R¹ Hydroxyl bedeutet, ausgeht und diese zunächst auf übliche Weise in eine aktivierte Form wie ein Halogenid, ein Anhydrid oder Imidazolid überführt und dieses dann mit einer entsprechenden Hydroxylverbindung HOR¹⁰ umsetzt. Diese Umsetzung läßt sich in den üblichen Lösungsmitteln durchführen und erfordert oft die Zugabe einer Base, wobei die oben genannten in Betracht kommen. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Außerdem können Verbindungen der Formel I auch dadurch hergestellt werden, daß man von den Salzen der entsprechenden Carbonsäuren ausgeht, d. h. von Verbindungen der Formel I, in denen R¹ für OM steht, wobei M ein Alkalimetallkation oder das Äquivalent eines Erdalkalimetallkations sein kann. Diese Salze lassen sich mit vielen Verbindungen der Formel R¹-A zur Reaktion bringen, wobei A eine übliche nucleofuge Abgangsgruppe bedeutet, beispielsweise Halogen wie Chlor, Brom, Iod oder gegebenenfalls durch Halogen, Alkyl oder Halogenalkyl substituiertes Aryl- oder Alkylsulfonyl wie z. B. Toluolsulfonyl und Methylsulfonyl oder eine andere äquivalente Abgangsgruppe. Verbindungen der Formel R¹-A mit einem reaktionsfähigen Substituenten A sind bekannt oder mit dem allgemeinen Fachwissen leicht zu erhalten. Diese Umsetzung läßt sich in den üblichen Lösungsmitteln durchführen und erfordert wieder oftmals die Zugabe einer Base, wobei die oben genannten in Betracht kommen.

Im Hinblick auf die biologische Wirkung sind 3-Aryloxy-Carbonsäurederivate der allgemeinen Formel I bevorzugt, in denen die Substituenten folgende Bedeutung haben:
- R¹: ein Rest OR¹⁰, worin R¹⁰ bedeutet:
Wasserstoff, das Kation eines Alkalimetalls oder das Kation eines Erdalkalimetalls wie Lithium, Natrium, Kalium, Calcium, Magnesium und Barium oder ein umweltverträgliches organisches Ammoniumion wie tert.-Alkylammoniumion mit bis zu 20 C-Atomen oder Ammmonium [NH₄^{⊕}];
C₁-C₁₀-Alkyl wie insbesonder Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl;
- R²: Methoxy bedeutet;
- X: CR¹⁴, worin
- R¹⁴: Wasserstoff bedeutet;
- R³: Methoxy bedeutet;
- R⁴: Phenyl, das durch einen oder mehrere der folgenden Reste substituiert sein kann: Halogen, Nitro, Cyano, Hydroxy, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, C₁-C₄-Alkylamino, C₁-C₄-Dialkylamino,
- R⁵: C₁-C₄-Alkyl;
- R⁶: Phenyl, das durch einen oder mehrere der folgenden Reste substituiert sein kann: Halogen, Nitro, Cyano, Hydroxy, Amino, C₁-C₄-Alkyl, C₁-C₄-Halogenalkyl, C₁-C₄-Alkoxy, C₁-C₄-Halogenalkoxy, Phenoxy, C₁-C₄-Alkylthio, C₁-C₄-Akylamino oder C₁-C₄-Dialkylamino;
- Y: Sauerstoff
- Z: Sauerstoff.

Besonders bevorzugt sind Verbindungen der Formel I, in der R² und R³ Methoxy und X CH bedeuten. Weiterhin bevorzugt sind Verbindungen der Formel I, in der R² und R³ Methoxy, X CH, Y und Z Sauerstoff und R⁵ C₁-C₄-Alkyl bedeuten. Bevorzugter Rest im Fall von R¹ ist die Gruppe OR¹⁰, wobei R¹⁰ Wasserstoff oder C₁-C₄-Alkyl bedeutet.

R⁴ steht besonders bevorzugt für gegebenenfalls substituiertes Phenyl.

R⁶ steht besonders bevorzugt für Phenyl, gegebenenfalls 1 - 3 fach substituiert durch Halogen, C₁-C₄-Alkyl und/oder Nitro.

Beispiele für bevorzugte Verbindungen sind in der nachfolgenden Tabelle aufgeführt.

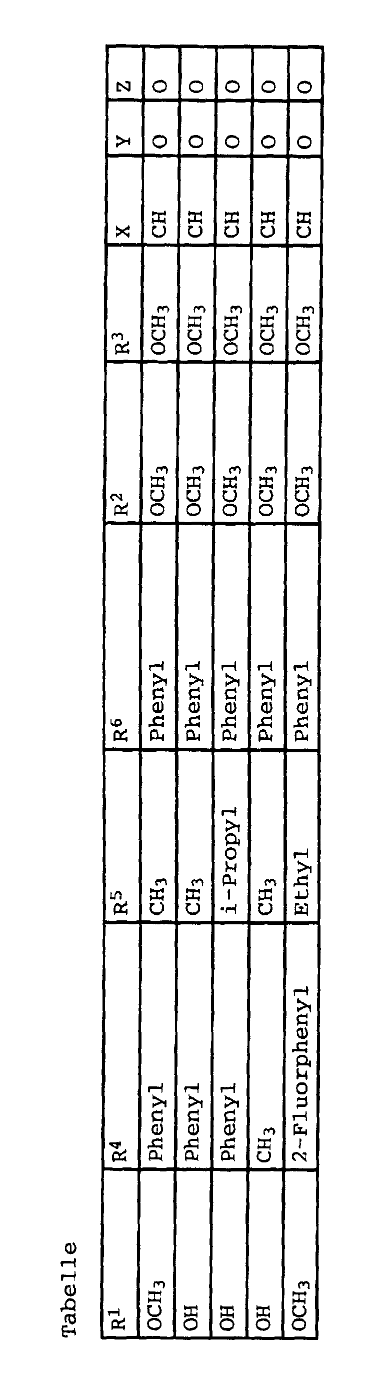

Die Verbindungen I bzw. die sie enthaltenden herbiziden Mittel sowie deren umweltvertragliche Salze, z.B. von Alkalimetallen und Erdalkalimetallen konnen in Kulturen wie Weizen, Reis, Mais, Soja und Baumwolle Schadpflanzen sehr gut bekampfen, ohne die Kulturpflanzen zu schädigen, ein Effekt, der vor allem auch bei niedrigen Aufwandmengen auftritt. Sie können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstauben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt verspruhbaren Lösungen, Emulsionen, Pasten oder Oldispersionen. Als inerte Zusatzstoffe kommen u.a. Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Waßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Ol bestehende Konzentrate hergestellt werden, die zur Verdunnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsaure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsauren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusol, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsauren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Tragerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 bis 100 %, vorzugsweise 95 bis 100 % (nach NMR-Spektrum) eingesetzt.

Beispiele für Formulierungen sind:
I. 20 Gewichtsteile der Verbindung Nr. 2.2 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsaure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
II. 20 Gewichtsteile der Verbindung Nr. 2.2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusol besteht. Durch Eingießen und feines Verteilen der Losung in 100 000 Gewichtsteilen Wasser erhalt man eine wäßrige Dispersion, die 0,02 Gew.% des Wirkstoffs enthalt.
III. 20 Gewichtsteile des Wirkstoffs Nr. 2.2 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusol besteht. Durch Eingießen und feines Verteilen der Lösung in 100 000 Gewichtsteilen Wasser erhalt man eine waßrige Dispersion, die 0,02 Gew.% des Wirkstoffs enthält.
IV. 20 Gewichtsteile des Wirkstoffs Nr. 2.2 werden mit 3 Gewichtsteilen des Natriumsalzes der Diisobutylnaphthalin-α-sulfonsaure, 17 Gewichtsteilen des Natriumsalzes einer Ligninsulfonsäure aus einer Sulfit-Ablauge und 60 Gewichtsteilen pulverförmigem Kieselsauregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
V. 3 Gewichtsteile des Wirkstoffs Nr. 2.2 werden mit 97 Gewichtsteilen feinteiligem Kaolin vermischt. Man erhält auf diese Weise ein Staubemittel, das 3 Gew.% des Wirkstoffs enthält.
VI. 20 Gewichtsteile des Wirkstoffs Nr. 2.2 werden mit 2 Gewichtsteilen Calciumsalz der Dodecylbenzolsulfonsaure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhalt eine stabile olige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgerate so gespritzt werden, daß die Blätter der empfindlichen Kulturpflanzen nach Möglichkeit nicht getroffen werden, während die Wirkstoffe auf die Blätter darunter wachsender unerwunschter Pflanzen oder die unbedeckte Bodenfläche gelangen (post-directed, lay-by).

Die Aufwandmengen an Wirkstoff betragen je nach Bekampfungsziel, Jahreszeit, Zielpflanzen und Wachstumsstadium 0,001 bis 5 kg/ha, vorzugsweise 0,01 bis 2 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwunschter Pflanzen eingesetzt werden. In Betracht kommen beispielsweise folgende Kulturen:

Allium cepa, Ananas comosus, Arachis hypogaea, Asparagus officinalis. Beta vulgaris spp. altissima, Beta vulgaris spp. rapa, Brassica napus var. napus, Brassica napus var. napobrassica, Brassica rapa var. silvescris, Camellia sinensis, Carthamus tinctorius, Carya illinoinensis, Citrus limon, Citrus sinensis, Coffea arabica (Coffea canephora, Coffea liberica), Coffea arabica (Coffea canephora, Coffea liberica), Cucumis sativus, Cynodon dactylon, Daucus carota, Elaeis guineensis, Fragaria vesca, Glycine max, Gossypium hirsutum (Gossypium arboreum, Gossypium herbaceum, Gossypium vitifolium), Gossypium hirsutum (Gossypium arboreum, Gossypium herbaceum, Gossypium vitifolium), Gossypium hirsutum (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (S. vulgare), Theobroma cacao, Trifolium pratense, Triticum aestivum, Triticum durum, Vicia faba, Vitis vinifera, Zea mays.

Die Verbindungen der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem
a) von der Pflanzenart und -sorte,
b) vom Zeitpunkt der Applikation, bezogen auf das Entwicklungsstadium der Pflanze und von der Jahreszeit,
c) von dem Applikationsort und -verfahren (z.B. Samenbeize, Bodenbehandlung, Blattapplikation oder Stamminjektion bei Bäumen),
d) von klimatischen Faktoren, z.B. Temperatur, Niederschlagsmenge, außerdem auch Tageslange und Lichtintensität,
e) von der Bodenbeschaffenheit (einschließlich Dungung),
f) von der Formulierung bzw. Anwendungsform des Wirkstoffs und schließlich
g) von der angewendeten Konzentration der aktiven Substanz.

Aus der Reihe der verschiedenartigen Anwendungsmöglichkeiten der Pflanzenwachstumsregulatoren der Formel I im Pflanzenanbau, in der Landwirtschaft und im Gartenbau, werden einige nachstehend erwähnt.
A. Mit den erfindungsgemäß verwendbaren Verbindungen läßt sich das vegetative Wachstum der Pflanzen stark hemmen, was sich ïnsbesondere in einer Reduzierung des Längenwachstums äußert.
   Die behandelten Pflanzen weisen demgemäß einen gedrungenen Wuchs aus; außerdem ist eine dunklere Blattfärbung zu beobachten.
   Als vorteilhaft für die Praxis erweist sich eine verminderte Intensität des Wachstums von Gräsern sowie lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollstandig mechanisiertes Beernten dieser wichtigen Kulturpflanze ermöglicht.
   Bei Obst- und anderen Bäumen lassen sich mit den Wachstumsregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu uppigen (und dadurch besonders frostanfälligen) Blatt- bzw.
   Pflanzenmasse gehemmt. Andererseits werden die jungen Rapspflanzen nach der Aussaat und vor dem Einsetzen der Winterfroste trotz gunstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide, ist es vorteilhaft, wenn die Bestände durch Behandlung mit den erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt bzw. Pflanzenmasse - dem Befall mit verschiedenen Krankheiten (z.B. Pilzkrankheit) vorgebeugt werden.
B. Mit den Wachstumsregulatoren lassen sich Mehrertrage sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Citrusfrüchten zu erhöhen, den Proteingehalt in Getreide oder Soja zu steigern oder Gummibäume zum vermehrten Latexfluß zu stimulieren.
   Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Citrusfrüchten, Oliven oder bei anderen Arten und Sorten von Kern-, Stein- und Schalenobst ermöglicht wird. Derselbe Mechanismus, d.h. die Förderung der Ausbildung von Trenngewebe zwischen Frucht-, bzw. Blatt- und Sproßteil der Pflanze ist auch für ein gut kontrollierbares Entblättern von Nutzpflanzen wie beispielsweise Baumwolle wesentlich.
D. Mit Wachstumsregulatoren kann weiterhin der Wasserverbrauch von Pflanzen reduziert werden. Durch den Einsatz der erfindungsgemäßen Substanzen läßt sich die Intensitat der Bewässerung reduzieren und damit eine kostengunstigere Bewirtschaftung durchführen, weil u.a.
   - die Öffnungsweite der Stomata reduziert wird,
   - eine dickere Epidermis und Cuticila ausgebildet werden,
   - die Durchwurzelung des Bodens verbessert wird und
   - das Mikroklima im Pflanzenbestand durch einen kompakteren Wuchs gunstig beeinflußt wird.

Besonders gut eignen sich sich Verbindungen I zur Halmverkürzung von Kulturpflanzen wie Gerste, Raps und Weizen.

Die erfindungsgemäß zu verwendenden Wirkstoffe der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Die Aufwandmenge an Wirkstoff ist infolge der hohen Pflanzenverträglichkeit nicht kritisch. Die optimale Aufwandmenge variiert je nach Bekämpfungsziel, Jahreszeit, Zielpflanzen und Wachstumsstadien.

Bei der Saatgutbehandlung werden im allgemeinen Wirkstoffmengen von 0.001 bis 50 g, vorzugsweise 0.01 bis 10 g, je Kilogramm Saatgut benötigt.

Für die Blatt- und Bodenbehandlung sind im allgemeinen Gaben von 0.001 bis 10 kg/ha, bevorzugt 0.01 bis 3 kg/ha, insbesondere 0.01 bis 0.5 kg/ha als ausreichend zu betrachten.

Zur Verbreiterung des Wirkungsspektrums und zur Erzielung synergistischer Effekte konnen die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy- bzw. Heteroaryloxy-phenoxypropionsauren sowie deren Salze, Ester und Amide und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekampfung von Schädlingen oder phytopathogenen Pilzen bzw. Bakterien. Von Interesse ist ferner die Mischbarkeit mit Mineralsalzlosungen, welche zur Behebung von Ernährungs- und Spurenelementmängeln eingesetzt werden. Es können auch nichtphytotoxische Öle und Ölkonzentrate zugesetzt werden.

### Synthesebeispiele

### Synthese von Verbindungen der allgemeinen Formel VI

### Beispiel 1

### 3-Phenoxy-3-phenyl-2-hydroxybuttersäuremethylester

28,2 g (0,3 mol) Phenol und 19,2 g (0,1 mol) 3-Phenyl-2,3-epoxybuttersauremethylester werden zusammen 6 Stunden auf 100°C erhitzt. Nach Abdestillieren des überschüssigen Phenols am Hochvakuum und chromatographischer Reinigung des Rückstands an Kieselgel mit Hexan/Essigestergemischen erhält man 17,9 g eines schwach gelben Öls.

Ausbeute: 62,5 %

### Beispiel 2

### 3-(4-Bromphenyl)oxy-3-phenyl-2-hydroxybuttersäuremethylester

51,9 g (0,3 mol) 4-Bromphenol und 19,2 g (0,1 mol)
3-Phenyl-2,3-epoxybuttersäuremethylester werden 8 h bei 100°C und 12 h bei Raumtemperatur gerührt. Nach Abdestillieren des überschüssigen Phenols wird der Rückstand mittels Flash-Chromatographie (Kieselgel, n-Hexan-Essigester 9:1) gereinigt. Man erhält 7,2 g eines weißen Feststoffes.

Ausbeute: 20 %
Fp.: 133 - 135°C
Analog wurden die in Tabelle 1 genannten Verbindungen hergestellt:

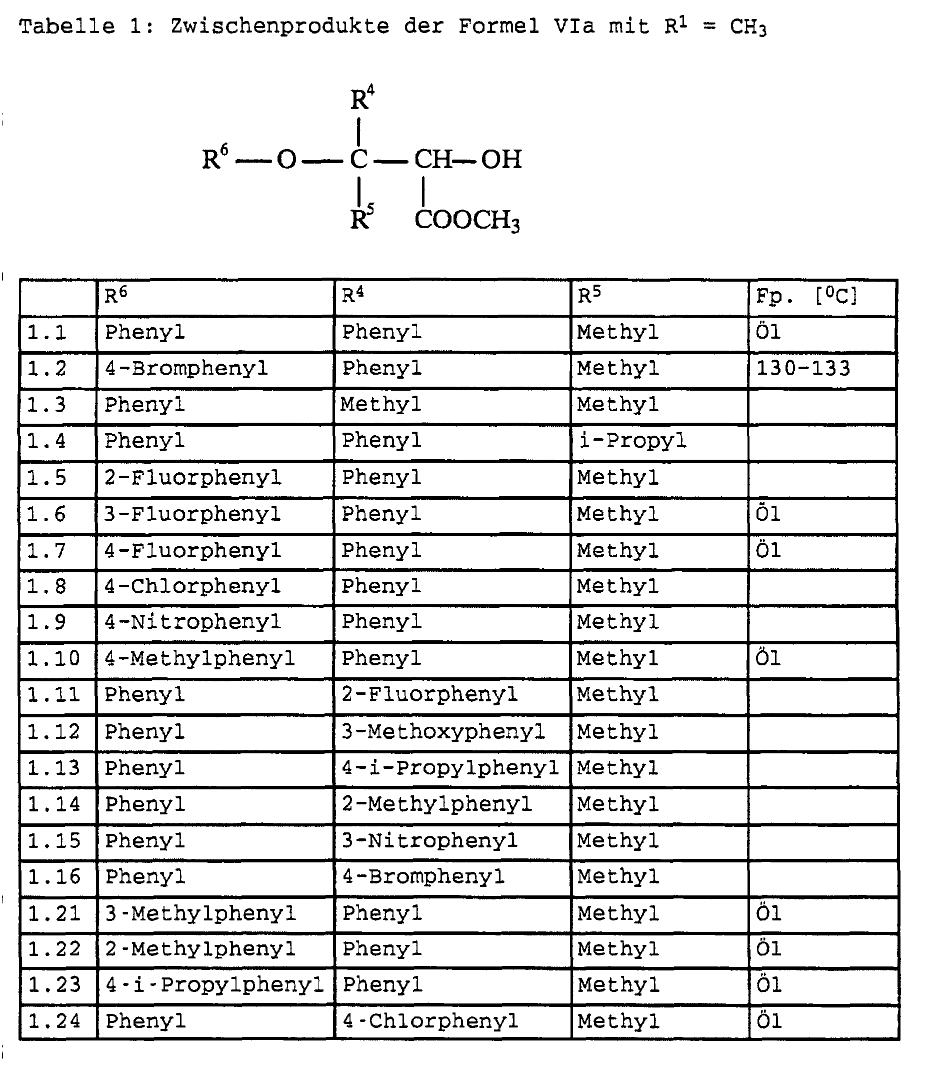

### Synthese von Verbindungen der allgemeinen Formel I:

### Beispiel 3

### 3-Phenoxy-3-phenyl-2-(4,6-dimethoxypyrimidin-2-yl)oxybuttersaure methylester

4,4 g (15,4 mmol) 3-Phenoxy-3-phenyl-2-hydroxybuttersauremethylester (Verb. 1.1) werden in 40 ml Dimethylformamid gelöst und mit 0,46 g (18,4 mmol) Natriumhydrid versetzt. Man rührt 1 Stunde und gibt dann 3,4 g (15,4 mmol) 4,6-Dimethoxy-2-methylsulfonylpyrimidin zu. Nach 24 Stunden Rühren bei Raumtemperatur wird vorsichtig mit 10 ml Wasser hydrolisiert, mit Essigsäure ein pH-Wert von 5 eingestellt und das Lösungsmittel am Hochvakuum abdestilliert. Der Rückstand wird in 100 ml Essigester aufgenommen, mit Wasser gewaschen, über Natriumsulfat getrocknet und das Lösungsmittel abdestilliert. Der Rückstand wird mit 10 ml Methyl-t-butylether versetzt und der gebildete Niederschlag abgesaugt. Nach dem Trocknen verbleiben 1,6 g eines weißen Pulvers.

Ausbeute: 24,5 %
Fp.: 143 - 145°C

### Beispiel 4

### 3-Phenoxy-3-phenyl-2-(4,6-dimethoxypyrimidin-2-yl)oxybuttersäure

1,3 g 3-Phenoxy-3-phenyl-2-(4,6-dimethoxypyrimidin-2-yl)oxybuttersauremethylester (Bsp. 3) werden in 20 ml MeOH und 40 ml Tetrahydrofuran gelöst und mit 3,7 g 10 % NaOH-Lösung versetzt. Man rührt 6 Stunden bei 60°C und 12 Stunden bei Raumtempertur, destilliert die Lösungsmittel im Vakuum ab und nimmt den Rückstand in 100 ml Wasser auf. Nicht umgesetzter Ester wird mit Essigester extrahiert. Anschließend stellt man die Wasserphase mit verdünnter Salzsaure auf pH 1 - 2 und extrahiert mit Essigester. Nach Trocknen über Magnesiumsulfat und Abdestillieren des Lösungsmittels verbleiben 1,0 g eines weißen Pulvers.

Ausbeute: 79,7 %
Fp.: 50 - 55°C

### Beispiel 5

### 3-Phenoxy-3-phenyl-2-[(4,6-dimethoxypyrmidin-2-yl)thio]buttersauremethylester

7,2 g (25 mmol) 3-Phenoxy-3-phenyl-2-hydroxybuttersauremethylester (Verb. 1.1) werden in 50 ml Dichlormethan gelost, 3 g (30 mmol) Triethylamin zugegeben und unter Ruhren 3,2 g (28 mmol) Methansulfonsaurechlorid zugetropft. Man rührt 2 Stunden bei Raumtemperatur, wascht mit Wasser, trocknet über Magnesiumsulfat und engt im Vakuum ein. Der Rückstand wird in 100 ml DMF aufgenommen und bei 0°C zu einer Suspension von 12,9 g (75 mmol) 4,6-Dimethoxpyrimidin-2-thiol und 8,4 g (100 mmol) Natriumhydrogencarbonat in 100 ml DMF getropft. Nach 2 Stunden Rühren bei Raumtemperatur und weiteren 2 Stunden bei 60°C gießt man auf 1 1 Eiswasser und saugt den entstandenen Niederschlag ab. Nach Trocknen verbleiben 4,2 g eines weißen Pulvers.

Ausbeute: 38 %

Analog den obigen Beispielen wurden die in Tabelle 2 genannten Verbindungen hergestellt.

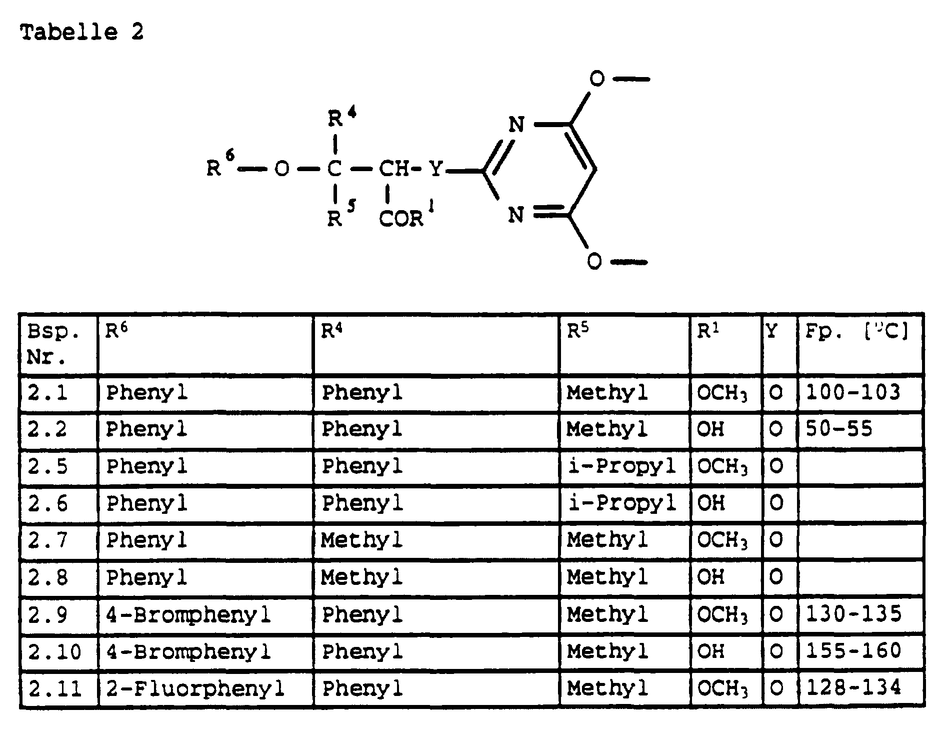

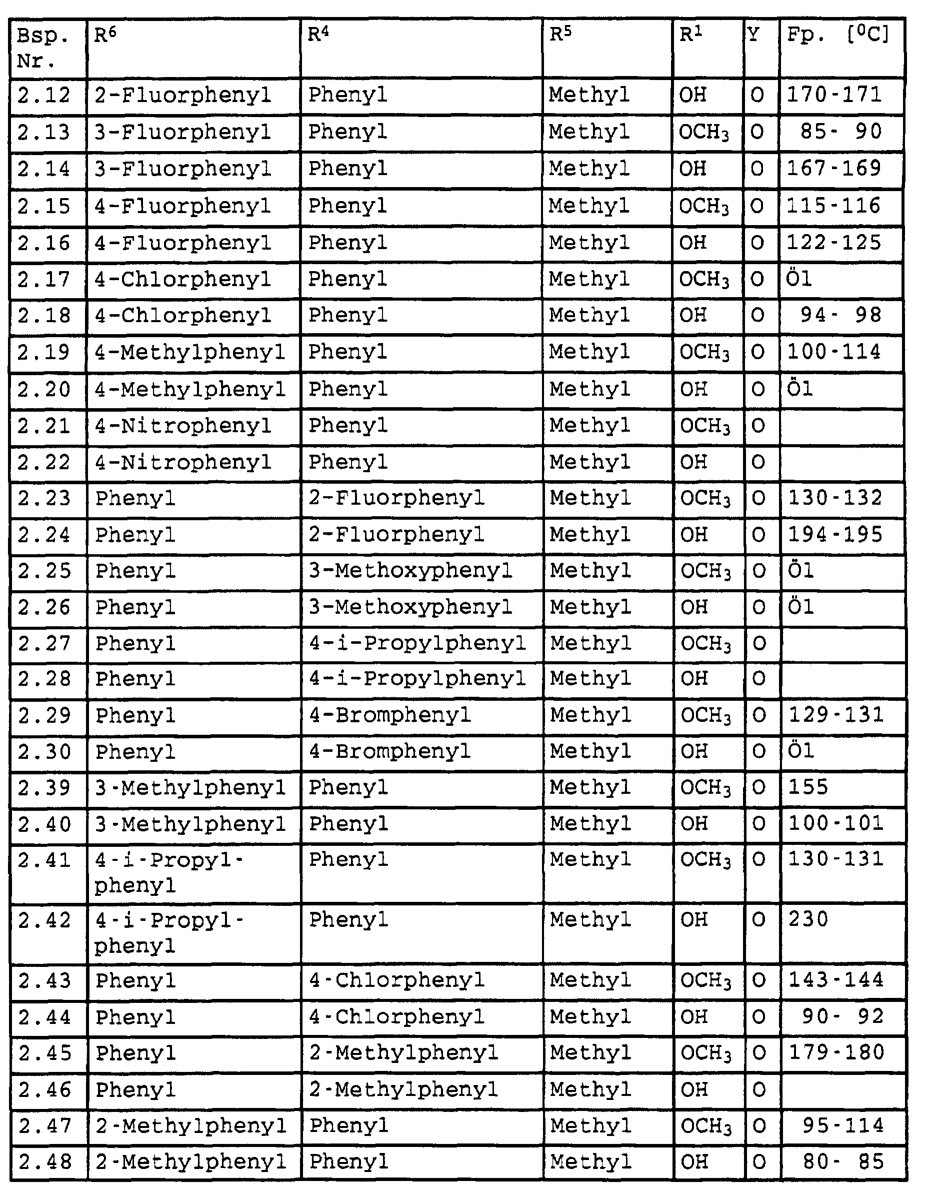

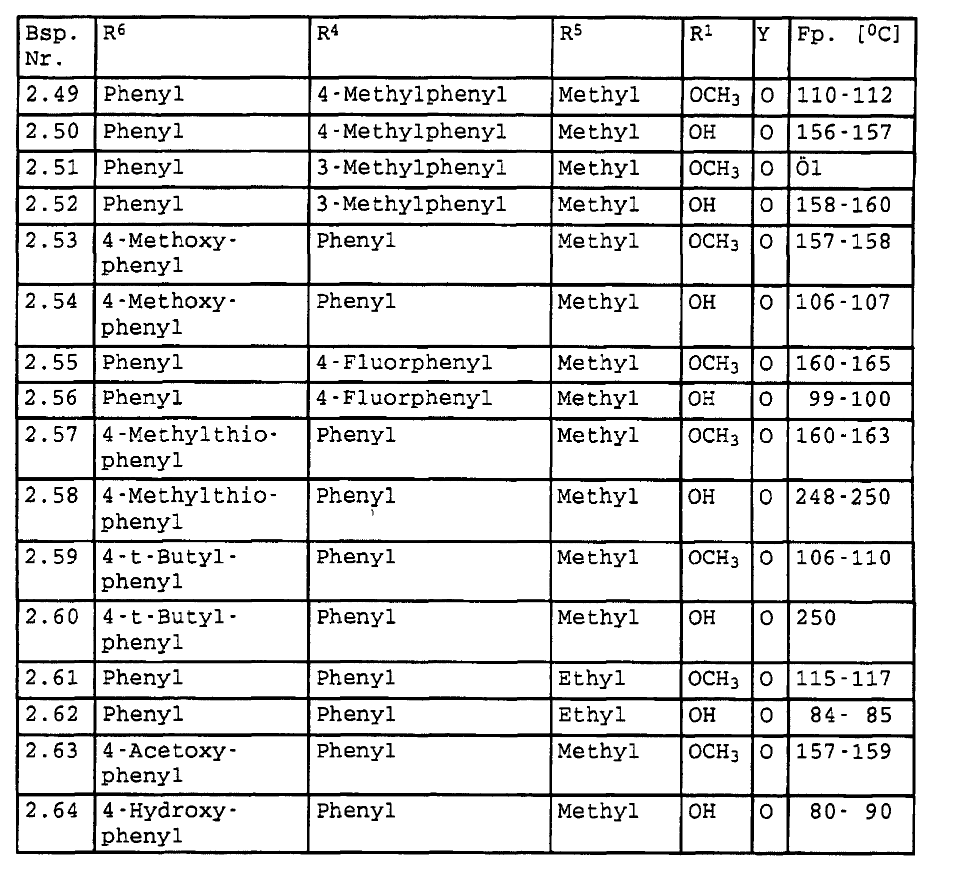

### Anwendungsbeispiele:

### a) Herbizide Wirkung

Die herbizide Wirkung der 3-Aryloxy-Carbonsäurederivate der Formel I wird durch folgende Gewächshausversuche gezeigt:

Als Kulturgefäße dienten Plastikblumentöpfe mit lehmigem Sand mit etwa 3,0 % Humus als Substrat. Die Samen der Testpflanzen werden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung werden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung beträgt 0,5 bzw. 0,25 kg/ha a.S. (aktive Substanz).

Die Pflanzen wurden artenspezifisch bei Temperaturen von 10 - 25°C bzw. 20 - 35°C gehalten. Die Versuchsperiode erstreckt sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name | Englischer Name |
|---|---|---|
| Amaranthus retroflexus | Zurückgekrümmter Fuchsschwanz | redroot pigweed |
| Polygonum persicaria | Flohknöterich | redshank |
| Solanum nigrum | Schwarzer Nachtschatten | black nightshade |

Mit 0,5 und 0,25 kg/ha a.S. im Nachauflaufverfahren eingesetzt lassen sich mit Beispiel Nr. 2.2 breitblättrige unerwünschte Pflanzen sehr gut bekämpfen.

### b) Bioregulatorische Wirkung

Die wachstumsregulierende Wirkung der 3-Aryloxy-Carbonsäurederivate der Formel I wird durch Längenmessung bestimmt, wobei die Anzucht und Behandlung der Testpflanzen wie voranstehend beschrieben vorgenommen wurde. Die Auswertung der Versuche erfolgte in der Weise, daß man die Wachshöhe der behandelten Pflanzen zur Wachshöhe von nicht behandelten Pflanzen in Beziehung setzte.

Die Versuchsergebnisse sind in den nachfolgenden Tabellen 3 - 7 zusammengestellt.

**Tabelle 3:**

| Sommerweizen "Ralle", Nachauflauf-Blattbehandlung | | |
|---|---|---|
| Beispiel-Nr. | Dosis kg/ha | Wuchshöhen relativ |
| unbehandelt | - | 100 |
| 2.17 | 0,1 | 88 |
| 2.19 | 0,1 | 94 |
| 2.64 | 0,1 | 89 |
| 2.59 | 0,5 | 76 |
| 2.55 | 0,5 | 82 |
| 2.50 | 0,5 | 89 |
| 2.62 | 0,5 | 78 |
| 2.60 | 0,5 | 83 |
| 2.61 | 0,5 | 81 |
| 2.56 | 0,5 | 77 |

**Tabelle 4:**

| Sommerweizen "Ralle", Nachauflauf-Blattbehandlung | | |
|---|---|---|
| Beispiel-Nr. | Dosis kg/ha | Wuchshöhen relativ |
| unbehandelt | - | 100 |
| 2.26 | 1,5 | 85 |
| | 0,75 | 92 |
| | 0,375 | 98 |
| | 0,1875 | 100 |
| 2.30 | 1,5 | 70 |
| | 0,75 | 79 |
| | 0,375 | 91 |
| | 0,1875 | - |
| 2.54 | 1,5 | 49 |
| | 0,75 | 64 |
| | 0,375 | 70 |
| | 0,1875 | 94 |
| 2.58 | 1,5 | 64 |
| | 0,75 | 76 |
| | 0,375 | 76 |
| | 0,1875 | 94 |
| 2.57 | 1,5 | 67 |
| | 0,75 | 67 |
| | 0,375 | 79 |
| | 0,1875 | 85 |
| 2.59 | 1,5 | 40 |
| | 0,75 | 40 |
| | 0,375 | 79 |
| | 0,1875 | 88 |
| 2.62 | 1,5 | 73 |
| | 0,75 | 73 |
| | 0,375 | 79 |
| | 0,1875 | 92 |
| 2.60 | 1,5 | 85 |
| | 0,75 | 89 |
| | 0,375 | 95 |
| | 0,1875 | 100 |

**Tabelle 5:**

| Sommergerste "Alexis", Nachauflauf-Blattbehandlung | | |
|---|---|---|
| Beispiel-Nr. | Dosis kg/ha | Wuchshöhen relativ |
| unbehandelt | - | 100 |
| 2.26 | 0,75 | 83 |
| | 0,375 | 86 |
| | 0,1875 | 96 |
| | 0,0937 | 96 |
| 2.30 | 1,5 | 63 |
| | 0,75 | 70 |
| | 0,375 | 83 |
| | 0,1875 | - |
| 2.54 | 1,5 | 43 |
| | 0,75 | 53 |
| | 0,375 | 66 |
| | 0,1875 | 86 |
| 2.58 | 1,5 | 76 |
| | 0,75 | 80 |
| | 0,375 | 100 |
| | 0,1875 | - |
| 2.57 | 1,5 | 60 |
| | 0,75 | 86 |
| | 0,375 | 86 |
| | 0,1875 | 86 |
| 2.59 | 1,5 | 70 |
| | 0,75 | 80 |
| | 0,375 | 90 |
| | 0,1875 | 93 |
| 2.62 | 1,5 | 73 |
| | 0,75 | 83 |
| | 0,375 | 83 |
| | 0,1875 | 87 |
| 2.42 | 0,5 | 84 |
| | 0,25 | 89 |
| | 0,125 | 95 |
| | 0,0625 | - |
| 2.59 | 0,5 | 86 |
| | 0,25 | 95 |
| | 0,125 | 95 |
| | 0,0625 | 95 |

**Tabelle 6:**

| Sommerraps "Petranova", Nachauflauf-Blattbehandlung | | |
|---|---|---|
| Beispiel-Nr. | Dosis kg/ha | Wuchshöhen relativ |
| unbehandelt | - | 100 |
| 2.17 | 0,1 | 74 |
| 2.19 | 0,1 | 77 |
| 2.39 | 0,1 | 77 |
| 2.63 | 0,1 | 92 |
| 2.64 | 0,1 | 90 |
| 2.51 | 0,5 | 69 |
| 2.23 | 0,5 | 69 |
| 2.43 | 0,5 | 66 |
| 2.53 | 0,5 | 69 |
| 2.44 | 0,5 | 69 |
| 2.57 | 0,5 | 83 |
| 2.55 | 0,5 | 60 |
| 2.50 | 0,5 | 72 |
| 2.62 | 0,5 | 81 |
| 2.60 | 0,5 | 72 |
| 2.61 | 0,5 | 96 |
| 2.56 | 0,5 | 87 |

**Tabelle 7:**

| Sommergerste "Alexis", Nachauflauf-Blattbehandlung | | |
|---|---|---|
| Beispiel-Nr. | Dosis kg/ha | Wuchshöhen relativ |
| unbehandelt | - | 100 |
| 2.26 | 0,75 | 57 |
| | 0,375 | 66 |
| | 0,1875 | 66 |
| | 0,0937 | 66 |
| 2.30 | 1,5 | - |
| | 0,75 | - |
| | 0,375 | 58 |
| | 0,1875 | 64 |
| 2.54 | 1,5 | 79 |
| | 0,75 | 79 |
| | 0,375 | 90 |
| | 0,1875 | 90 |
| 2.58 | 1,5 | 85 |
| | 0,75 | - |
| | 0,375 | 85 |
| | 0,1875 | 85 |
| 2.42 | 0,5 | 55 |
| | 0,25 | 55 |
| | 0,125 | 68 |
| | 0,0625 | 68 |
| 2.59 | 0,5 | 79 |
| | 0,25 | 79 |
| | 0,125 | 84 |
| | 0,0625 | 84 |

## Patentansprüche

1. 3-Aryloxy-Carbonsäure-derivate der allgemeinen Formel I, 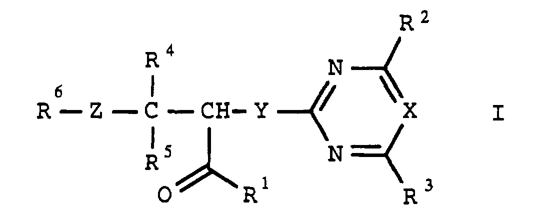 in der die Substituenten folgende Bedeutung haben:
R¹ einen Rest OR¹⁰, worin R¹⁰ bedeutet:
i) Wasserstoff, ein Alkalimetallkation, das Äquivalent eines Erdalkalimetallkations, das Ammoniumkation oder ein organisches Ammoniumion;
ii) eine C₁-C₁₀-Alkylgruppe;
R² Methoxy;
X CR¹⁴, wobei R¹⁴ Wasserstoff bedeutet;
R³ Methoxy;
R⁴ Phenyl, das durch einen oder mehrere der folgenden Reste substituiert sein kann: Halogen, Nitro, Cyano, Hydroxy, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, C₁-C₄-Alkylamino oder C₁-C₄-Dialkylamino;
R⁵ C₁-C₄-Alkyl;
R⁶ Phenyl, das durch einen oder mehrere der folgenden Reste substituiert sein kann: Halogen, Nitro, Cyano, Hydroxy, Amino, C₁-C₄-Alkyl, C₁-C₄-Halogenalkyl, C₁-C₄-Alkoxy, C₁-C₄-Halogenalkoxy, Phenoxy, C₁-C₄-Alkylthio, C₁-C₄-Alkylamino oder C₁-C₄-Dialkylamino;
Y Sauerstoff;
Z Sauerstoff.

2. Aryloxy-Carbonsäurederivate der Formel I gemäß Anspruch 1, in denen Y und Z Sauerstoff, X CH, R² und R³ Methoxy, R⁵ Methyl, R⁴ und R⁶ Phenyl, das wie in Anspruch 1 genannt substituiert sein kann, bedeuten und R¹ die in Anspruch 1 genannte Bedeutung hat.

3. Herbizides Mittel, enthaltend eine Verbindung der Formel I gemäß den Ansprüchen 1 und 2 und übliche inerte Zusatzstoffe.

4. Verfahren zur Bekämpfung unerwünschten Pflanzenwuchses, **dadurch gekennzeichnet, daß** man eine herbizid wirksame Menge einer Verbindung der Formel I gemäß Anspruch 1 auf die Pflanzen oder deren Lebensraum einwirken läßt.

5. Mittel zur Beeinflussung des Pflanzenwachstums, enthaltend eine Verbindung der Formel I gemäß Anspruch 1 und übliche inerte Zusatzstoffe.

6. Verfahren zur Regulierung des Pflanzenwachstums, **dadurch gekennzeichnet, daß** man eine bioregulatorisch wirksame Menge einer Verbindung der Formel I gemäß Anspruch 1 auf die Pflanzen oder deren Lebensraum einwirken läßt.

7. Verfahren zur Herstellung von 3-Aryloxycarbonsäurederivaten der allgemeinen Formel I gemäß Anspruch 1, **dadurch gekennzeichnet, daß** man 3-Aryloxy-Carbonsäurederivate der allgemeinen Formel VI 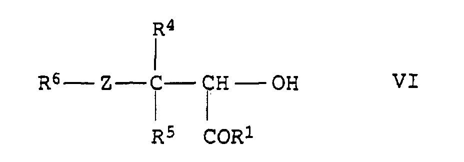 in der R¹, R⁴, R⁵, R⁶ und Z die in Anspruch 1 genannte Bedeutung haben, mit Verbindungen der allgemeinen Formel VII, 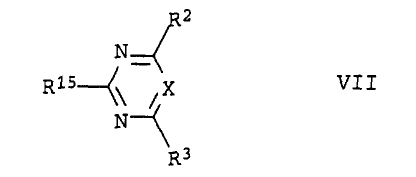 in der R¹⁵ Halogen oder R¹⁶SO₂- bedeutet, wobei R¹⁶ C₁-C₄-Alkyl, C₁-C₄-Halogenalkyl oder Phenyl ist, in einem inerten Lösungsmittel unter Zugabe einer Base umsetzt.

8. Verfahren zur Herstellung von 3-Aryloxy-Carbonsäurederivaten der allgemeinen Formel I gemäß Anspruch 1, wobei Y Schwefel bedeutet, **dadurch gekennzeichnet, daß** man 3-Aryloxy-Carbonsäurederivate der allgemeinen Formel VIII, 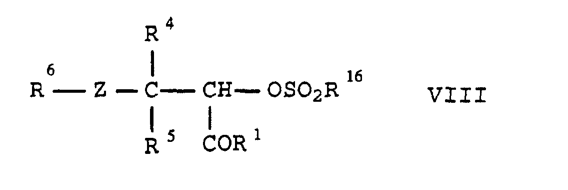 in der die Substituenten die in Anspruch 7 angegebene Bedeutung haben, mit Verbindungen der allgemeinen Formel IX, 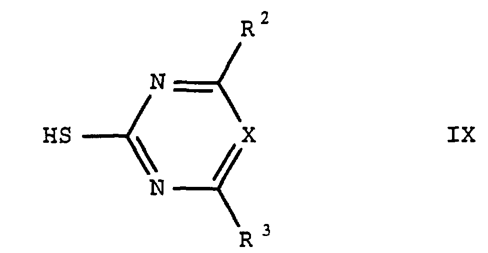 in der die Substituenten die in Anspruch 1 genannte Bedeutung haben, zur Reaktion bringt.

## Claims

1. A 3-aryloxycarboxylic acid derivative of the formula I 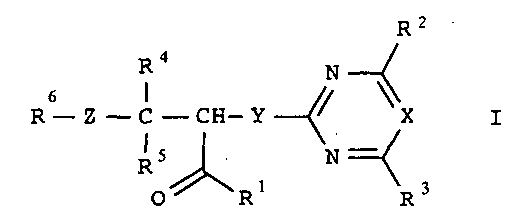 where
R¹ is a radical OR¹⁰, where R¹⁰ is:
i) hydrogen, an alkali metal cation, one equivalent of an alkaline earth metal cation, the ammonium cation or an organic ammonium ion;
ii) C₁-C₁₀-alkyl;
R² is methoxy;
X is CR¹⁴, where R¹⁴ is hydrogen;
R³ is methoxy;
R⁴ is phenyl, which may be substituted by one or more of the following radicals: halogen, nitro, cyano, hydroxyl, C₁-C₄-alkyl, C₁-C₄-haloalkyl, C₁-C₄-alkoxy, C₁-C₄-haloalkoxy, phenoxy, C₁-C₄-alkylthio, amino, C₁-C₄-alkylamino or C₁-C₄-dialkylamino;
R⁵ is C₁-C₄-alkyl;
R⁶ is phenyl, which may be substituted by one or more of the following radicals: halogen, nitro, cyano, hydroxyl, amino, C₁-C₄-alkyl, C₁-C₄-haloalkyl, C₁-C₄-alkoxy, C₁-C₄-haloalkoxy, phenoxy, C₁-C₄-alkylthio, C₁-C₄-alkylamino or C₁-C₄-dialkylamino;
Y is oxygen; and
Z is oxygen.

2. An aryloxycarboxylic acid derivative of the formula I as claimed in claim 1, in which Y and Z are each oxygen, X is CH, R² and R³ are each methoxy, R⁵ is methyl, R⁴ and R⁶ are each phenyl which may be substituted as stated in claim 1 and R¹ has the meanings stated in claim 1.

3. A herbicide containing a compound of the formula I as claimed in claims 1 and 2 and conventional inert additives.

4. A method for controlling undesirable plant growth, wherein a herbicidal amount of a compound of the formula I as claimed in claim 1 is allowed to act on the plants or on their habitat.

5. An agent for influencing plant growth, containing a compound of the formula I as claimed in claim 1 and conventional inert additives.

6. A method for regulating plant growth, wherein a bioregulatory amount of a compound of the formula I as claimed in claim 1 is allowed to act on the plants or on their habitat.

7. A process for the preparation of 3-aryloxycarboxylic acid derivatives of the formula I as claimed in claim 1, wherein a 3-aryloxycarboxylic acid derivative of the formula VI 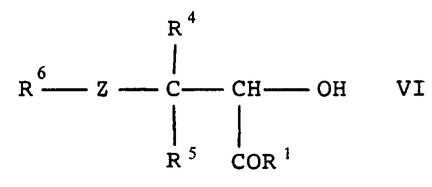 where R¹, R⁴, R⁵, R⁶ and Z have the meanings stated in claim 1, is reacted with a compound of the formula VII 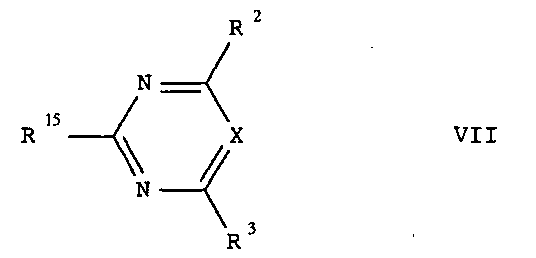 where R¹⁵ is halogen or R¹⁶SO₂- and R¹⁶ is C₁-C₄-alkyl, C₁-C₄-haloalkyl or phenyl, in an inert solvent with the addition of a base.

8. A process for the preparation of a 3-aryloxycarboxylic acid derivative of the formula I as claimed in claim 1, where Y is sulfur, wherein a 3-aryloxycarboxylic acid derivative of the formula VIII 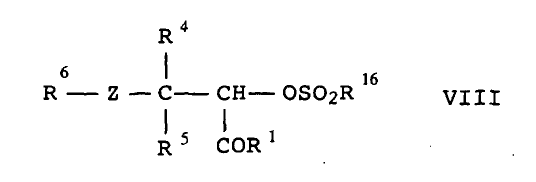 where the substituents have the meanings stated in claim 7, is reacted with a compound of the formula IX 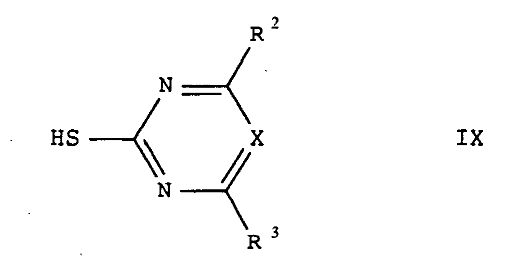 where the substituents have the meanings stated in claim 1.

## Revendications

1. Dérivés d'acides 3-aryloxy-carboxyliques de formule générale I 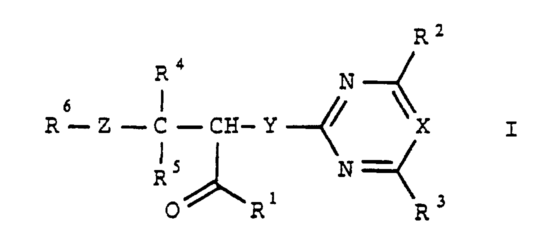 dans laquelle les symboles ont les significations suivantes :
R¹ représente un groupe OR¹⁰ dans lequel R¹⁰ représente
i) l'hydrogène, un cation de métal alcalin, un équivalent d'un cation de métal alcalino-terreux, le cation ammonium ou un ion ammonium organique ;
ii) un groupe alkyle en C1-C10 ;
R² représente un groupe méthoxy ;
X représente le groupe CR¹⁴ dans lequel R¹⁴ représente l'hydrogène ;
R³ représente un groupe méthoxy ;
R⁴ représente un groupe phényle qui peut porter un ou plusieurs des substituants suivants : halogéno, nitro, cyano, hydroxy, alkyle en C1-C4, halogénoalkyle en C1-C4, alcoxy en C1-C4, halogénoalcoxy en C1-C4, phénoxy, alkylthio en C1-C4, amino, alkylamino en C1-C4 ou di-(alkyle en C1-C4)amino ;
R⁵ représente un groupe alkyle en C1-C4 ;
R⁶ représente un groupe phényle qui peut porter un ou plusieurs des substituants suivants : halogéno, nitro, cyano, hydroxy, amino, alkyle en C1-C4, halogénoalkyle en C1-C4, alcoxy en C1-C4, halogénoalcoxy en C1-C4, phénoxy, alkylthio en C1-C4, alkylamino en C1-C4 ou di-(alkyle en C1-C4)amino ;
Y représente l'oxygène ;
Z représente l'oxygène.

2. Dérivés d'acides aryloxy-carboxyliques de formule I de la revendication 1, dans laquelle Y et Z représentent l'oxygène, X représente CH, R² et R³ des groupes méthoxy, R⁵ un groupe méthyle, R⁴ et R⁶ des groupes phényle pouvant porter les substituants indiqués dans la revendication 1 et R¹ a les significations indiquées dans la revendication 1.

3. Produit herbicide contenant un composé de formule I selon les revendications 1 et 2 et des additifs inertes usuels.

4. Procédé pour combattre les croissances de végétaux indésirables, **caractérisé par le fait que** l'on fait agir une quantité herbicide efficace d'un composé de formule I de la revendication 1 sur les végétaux ou leur habitat.

5. Produit pour agir sur la croissances des végétaux, contenant un composé de formule I de la revendication 1 et des additifs inertes usuels.

6. Procédé pour la régulation de la croissance des végétaux, **caractérisé par le fait que** l'on fait agir une quantité biorégulatrice efficace d'un composé de formule I de la revendication 1 sur les végétaux ou leur habitat.

7. Procédé pour la préparation des dérivés d'acides 3-aryloxy-carboxyliques de formule générale I de la revendication 1, **caractérisé par le fait que** l'on fait réagir des dérivés d'acides 3-aryloxycarboxyliques de formule générale VI 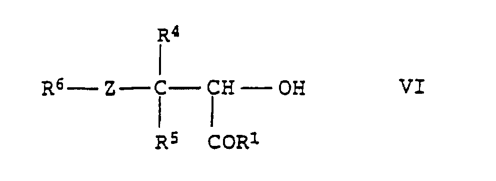 dans laquelle R¹, R⁴, R⁵, R⁶ et Z ont les significations indiquées dans la revendication 1, avec des composés de formule générale VII 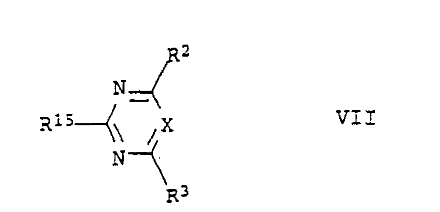 dans laquelle R¹⁵ représente un halogène ou le groupe R¹⁶SO₂- dans lequel R¹⁶ représente un groupe alkyle en C1-C4, halogénoalkyle en C1-C4 ou phényle, dans un solvant inerte et en présence d'une base.

8. Procédé pour la préparation des dérivés d'acides 3-aryloxy-carboxyliques de formule générale I de la revendication 1 dans laquelle Y représente le soufre, **caractérisé par le fait que** l'on fait réagir des dérivés d'acides 3-aryloxy-carboxyliques de formule générale VIII 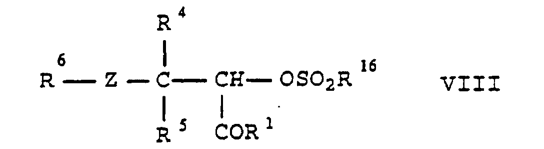 dans laquelle les symboles ont les significations indiquées dans la revendication 7, avec des composés de formule générale IX 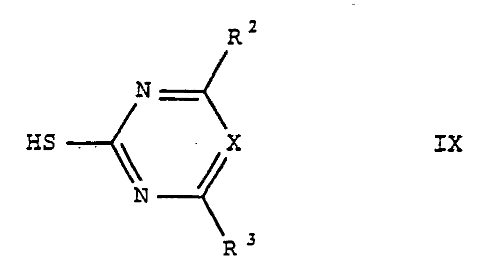 dans laquelle les symboles ont les significations indiquées dans la revendication 1.
